# EUROPÄISCHE PATENTSCHRIFT

(11) **EP 0 590 446 B1**
(45) Veröffentlichungstag und Bekanntmachung des Hinweises auf die Patenterteilung: **21.05.1997**
(21) Anmeldenummer: 93115037.9
(22) Anmeldetag: 18.09.1993
(51) Int. Cl.: C07D 487/04, G03G 9/08

(54) **Benzimidazole und ihre Anwendung als Ladungsstabilisatoren**
Benzimidazoles and their use as charge stabilizers
Benzimidazoles et leur utilisation comme stabilisateurs de la charge

(30) Priorität: 29.09.1992 DE 4232524
(43) Veröffentlichungstag der Anmeldung: 06.04.1994
(73) Patentinhaber: BASF Aktiengesellschaft, 67063 Ludwigshafen (DE)
(72) Erfinder: Schroeder, Gunter-Rudolf, Dr., D-6900 Heidelberg (DE); Mayer, Udo, Dr., D-6710 Frankenthal (DE)

(56) Entgegenhaltungen:
- EP-A- 0 347 695
- DE-A- 2 733 468
- PATENT ABSTRACTS OF JAPAN vol. 16, no. 431 (C-983) (5474) 9. September 1992 & JP-A-04 149 180 (FUJI PHOTO FILM CO LTD) 22. Mai 1992

## Beschreibung

Die vorliegende Erfindung betrifft neue Benzimidazole der Formel I 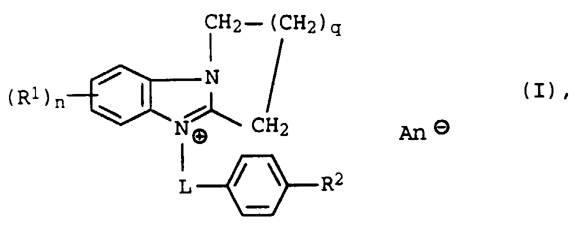 in der
- n und q: unabhängig voneinander jeweils 1 oder 2,
- R¹: Wasserstoff, Chlor oder Methyl,
- R²: Wasserstoff, gegebenenfalls durch Hydroxy oder C₁-C₄-Alkoxy substituiertes C₁-C₄-Alkyl, Halogen, Nitro oder C₁-C₄-Alkanoyl,
- L: C₂-C₆-Alkylen und
- An^{⊖}: das Äquivalent eines Anions bedeuten,
elektrostatische Toner, enthaltend die Benzimidazole als Ladungsstabilisatoren sowie die Verwendung der Benzimidazole als Ladungsstabilisatoren.

Aus der DE-A-2 733 468 und der US-A-4 912 006 sind bereits ähnliche Benzimidazolverbindungen bekannt. Es hat sich jedoch gezeigt, daß sie bei ihrer Anwendung als Ladungsstabilisatoren in elektrostatischen Tonern noch anwendungstechnische Mängel aufweisen.

Aufgabe der vorliegenden Erfindung war es nun, neue Benzimidazole bereitzustellen, die über ein vorteilhaftes anwendungstechnisches Eigenschaftsprofil verfügen.

Demgemäß wurden die eingangs näher bezeichneten Benzimidazole der Formel I gefunden.

Alle in Formel I auftretenden Alkyl- oder Alkylengruppen können sowohl geradkettig als auch verzweigt sein.

Reste R² sind z.B. Methyl, Ethyl, Propyl, Isopropyl, Butyl, Isobutyl, sec-Butyl, 2-Hydroxyethyl, 2- oder 3-Hydroxypropyl, 2- oder 4-Hydroxybutyl, 2-Methoxyethyl, 2- oder 3-Methoxypropyl, 2- oder 4-Methoxybutyl, 2-Ethoxyethyl, 2- oder 3-Ethoxypropyl, 2- oder 4-Ethoxybutyl, Fluor, Chlor, Brom, Formyl, Acetyl, Propionyl, Butyryl oder Isobutyryl.

Reste L sind z.B. (CH₂)₂, (CH₂)₃, (CH₂)₄, (CH₂)₅, (CH₂)₆, CH(CH₃)CH₂ oder CH(CH₃)CH(CH₃).

Geeignete Anionen sind z.B. anorganische oder organische Anionen, beispielsweise Halogenide, wie Fluorid, Chlorid, Bromid oder Iodid, Hexafluorophosphat, Tetrafluoroborat, Formiat, Acetat, Propionat, Oxalat, Benzolsulfonat, Toluolsulfonat oder Tetraphenylboranat.

Bevorzugt sind Benzimidazole der Formel I, in der
- q: 1,
- R¹: Wasserstoff,
- R²: Wasserstoff oder C₁-C₄-Alkyl und
- L: C₃-C₆-Alkylen bedeuten und
- n und An^{⊖}: jeweils die oben genannte Bedeutung besitzen.

Von besonderem Interesse sind Benzimidazole der Formel I, in der L C₃-C₄-Alkylen bedeutet.

Besonders hervorzuheben ist das Benzimidazol der Formel Ia 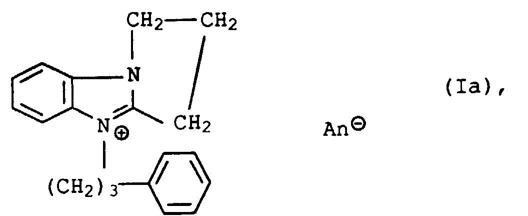 in der An^{⊖} die obengenannte Bedeutung besitzt.

Die erfindungsgemäßen Benzimidazole der Formel I können nach an sich bekannten Methoden, wie sie beispielsweise in der DE-A-2 733 468 beschrieben sind, erhalten werden.

So kann man ein verbrücktes Benzimidazol der Formel II 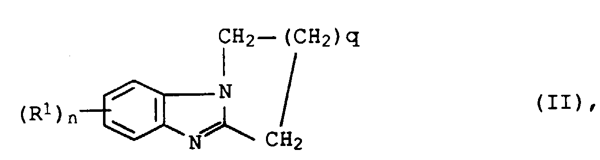 in der n, q und R¹ jeweils die obengenannte Bedeutung besitzen, mit einer Verbindung der Formel III 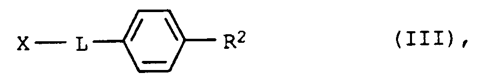 in der L und R² jeweils die obengenannte Bedeutung besitzen und X für eine Austrittsgruppe, z.B. Chlor, Brom oder Iod, steht, umsetzen und gegebenenfalls anschließend mittels eines Salzes der Formel IV

M^{⊕} An^{⊖} (IV),

in der An^{⊖} die obengenannte Bedeutung besitzt und M^{⊕} für das Äquivalent eines Metallkations, z.B. Natrium oder Kalium, steht, ausfällen.

Die neuen Benzimidazole eignen sich vorteilhaft als Ladungsstabilisatoren in elektrostatischen Tonern.

Demgemäß betrifft die vorliegende Erfindung weiterhin elektrostatische Toner, enthaltend ein polymeres Bindemittel und als Ladungsstabilisator ein Benzimidazol der Formel I.

Der Anteil der Benzimidazole der Formel I im elektrostatischen Toner beträgt in der Regel 0,01 bis 10 Gew.-%, bezogen auf das Gewicht des Toners.

Die in den neuen elektrostatischen Tonern enthaltenen polymeren Bindemittel sind an sich bekannt. Sie sind in der Regel thermoplastisch und haben einen Erweichungspunkt von 40 bis 200°C, vorzugsweise 50 bis 130°C und insbesondere 65 bis 115°C. Beispiele für polymere Bindemittel sind Polystyrol, Copolymere von Styrol mit einem Acrylat oder Methacrylat, Copolymere von Styrol mit Butadien und/oder Acrylnitril, Polyacrylate, Polymethacrylate, Copolymere eines Acrylats oder Methacrylats mit Vinylchlorid oder Vinylacetat, Polyvinylchlorid, Copolymere von Vinylchlorid mit Vinylidenchlorid, Copolymere von Vinylchlorid mit Vinylacetat, Polyesterharze, Epoxyharze, Polyamide oder Polyurethane.

Zusätzlich zu den obengenannten Benzimidazolen I und den polymeren Bindemitteln können die erfindungsgemäßen Toner in bekannten Mengen Farbmittel, magnetisch anziehbares Material, Wachse und Fließmittel enthalten.

Die Farbmittel können organische Farbstoffe oder Pigmente, wie Nigrosin, Anilinblau, 2,9-Dimethylchinacridon, C.I. Disperse Red 15 (C.I. 6010), C.I. Solvent Red 19 (C.I. 26 050), C.I. Pigment Blue 15 (C.I. 74 160), C.I. Pigment Blue 22 (C.I. 69 810) oder C.I. Solvent Yellow 16 (C.I. 12 700) oder anorganische Pigmente, wie Ruß, Rotblei, gelbes Bleioxid oder Chromgelb, sein. Allgemein überschreitet die Menge des im Toner vorhandenen Farbmittels nicht 15 Gew.-%, bezogen auf das Gewicht des Toners.

Das magnetisch anziehbare Material kann beispielsweise Eisen, Nickel, Chromoxid, Eisenoxid oder ein Ferrit der Formel MeFe₂O₄, worin Me ein zweiwertiges Metall, z.B. Eisen, Kobalt, Zink, Nickel oder Mangan, darstellt, sein.

Die Herstellung der erfindungsgemäßen Toner erfolgt nach üblichen Verfahren, z.B. durch Vermischen der Bestandteile in einem Kneter und anschließendes Pulverisieren oder durch Schmelzen des polymeren Bindemittels oder eines Gemisches der polymeren Bindemittel, anschließende feine Zerteilung eines oder mehrerer Benzimidazole I, sowie der anderen Zusätze, falls verwendet, in dem geschmolzenen Harz unter Anwendung der für diesen Zweck bekannten Misch- und Knetmaschinen, anschließende Abkühlung der Schmelze zu einer festen Masse und schließlich Vermahlen der festen Masse zu Teilchen der gewünschten Teilchengröße (in der Regel 0,1 bis 50 µm). Es ist auch möglich, das polymere Bindemittel und den Ladungsstabilisator in einem gemeinsamen Lösungsmittel zu suspendieren und die anderen Zusätze in die Suspension zu geben. Die Suspension kann so als Flüssigtoner verwendet werden.

Man kann die Flüssigkeit aber auch in an sich bekannter Weise sprühtrocknen, die Lösungsmittel abdampfen oder die Flüssigkeit gefriertrocknen und den festen Rückstand zu Teilchen der gewünschten Teilchengröße vermahlen.

Es ist weiterhin möglich, die als Ladungstabilisatoren verwendeten neuen Benzimidazole nicht zu lösen, sondern fein in der Lösung des polymeren Bindemittels zu dispergieren. Die so erhaltene Tonerzubereitung kann dann, beispielsweise gemäß der US-A-4 265 990, in einem xerographischen Bildaufzeichnungssystem verwendet werden.

Die obengenannten Benzimidazole der Formel I sind vorteilhafte Ladungsstabilisatoren. Sie zeichnen sich besonders dadurch aus, daß sie bei Zusatz zu einer Tonerpräparation dieser ein günstiges elektrostatisches Aufladungsprofil verleihen, d.h. die Toner lassen sich schnell und hoch aufladen. Die erfindungsgemäßen Ladungsstabilisatoren bewirken weiterhin, daß die Ladung auf einem hohen Niveau konstant gehalten wird.

Die folgenden Beispiele sollen die Erfindung näher erläutern.

### A) Herstellung der Benzimidazole

### Beispiel H1

79 g Pyrrolidino[1,2-a]benzimidazol und 99,6 g 1-Brom-3-phenylpropan wurden 4 Stunden bei 140°C gerührt. Die heiße Schmelze wurde dann in 1300 ml Wasser eingetragen, wobei eine klare dunkle Lösung entstand. Bei 60°C wurden 60,4 g Natriumtetrafluoroborat zugegeben. Der entstandene Niederschlag wurde abfiltriert, aus Isopropanol umkristallisiert und getrocknet. Man erhielt 159,5 g der Verbindung der Formel 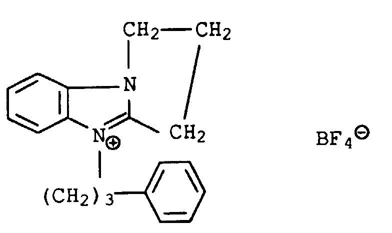
- Analyse:: C 62,6 % (ber. 62,6 %)
H 5,9 % (ber. 5,8 % )
N 7,7 % (ber. 7,7 %)

In analoger Weise werden die folgenden Benzimidazole der Formel 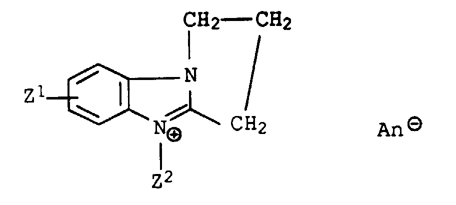 erhalten.

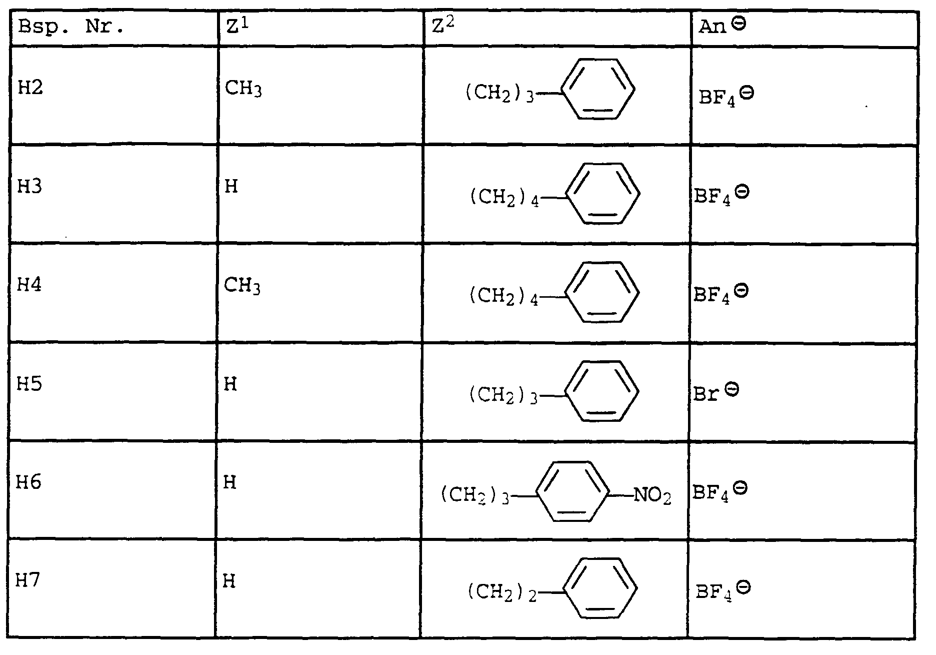

### B) Anwendung

Die Anwendungsbeispiele wurden mit farbmittelfreien Tonermodellen, bestehend aus Harz und den erfindungsgemäßen Ladungsstabilisatoren, durchgeführt.

### I. Herstellung der Toner

### Beispiel A1

In eine Lösung von 10 g eines nicht vernetzten Styrol/Butylacrylatharzes in 100 ml Xylol wurden bei Raumtemperatur 0,2 g des Benzimidazols aus Beispiel H 1 eingetragen und anschließend gefriergetrocknet.

### Beispiel A2

In einem Mixer wurden 10 g eines nicht vernetzten Styrol/Butylacrylatharzes und 0,2 g des Benzimidazols aus Beispiel H 1 intensiv gemischt, bei 120°C geknetet, extrudiert und gemahlen. Es wurden durch Sichtung Tonerteilchen einer mittleren Partikelgröße von 15 µm erzeugt.

### II. Herstellung der Developer und Prüfung

Zur Herstellung eines Developers wurden 99 Gew.-% eines Stahlcarriers, der eine mittlere Teilchengröße von 50 µm aufwies, mit 1 Gew.-% des Toners genau eingewogen und über einen unten näher bestimmten Zeitraum auf einem Rollenbock aktiviert. Danach wurde die elektrostatische Aufladung des Developers bestimmt. Etwa 5 g des aktivierten Developers wurden in einem handelsüblichen q/m Meter (Firma Epping GmbH, Neufahrn) in eine hard-blow-off-Zelle, die mit einem Elektrometer elektrisch verbunden war, eingefüllt. Die Maschenweiten der in der Meßzelle eingesetzten Siebe betrug 50 µm.

Damit war gewährleistet, daß der Toner möglichst vollständig ausgeblasen wurde, der Carrier aber in der Meßzelle verblieb. Durch einen kräftigen Luftstrom (ca. 4 000 cm³/min) und gleichzeitigem Absaugen wurde der Toner nahezu vollständig von den Carrierteilchen entfernt, wobei letztere in der Meßzelle verblieben. Die Aufladung des Carriers wurde am Elektrometer registriert. Sie entsprach dem Betrag der Aufladung der Tonerteilchen, nur mit umgekehrten Vorzeichen. Zur Berechnung des q/m-Wertes wurde deshalb der Betrag von q mit den umgekehrten Vorzeichen verwendet. Durch Zurückwiegen der Meßzelle wurde die Masse an ausgeblasenem Toner bestimmt und daraus die elektrostatische Aufladung q/m berechnet.

Die an den Tonern bestimmte Aufladung ist in der folgenden Tabelle zusammengefaßt.

**Tabelle**

| Beispiel Nr. | Verbindung aus Beispiel | Aufladung nach einer Aktivierung von | | | |
|---|---|---|---|---|---|
| | | 10 min | 30 min | 60 min | 120 min |
| | | [µC/g] | | | |
| A1 | H1 | 6,1 | 5,7 | 5,6 | 5,2 |
| A2 | H2 | 6,5 | 6,0 | 6,0 | 5,7 |
| A3 | H3 | 5,2 | 5,0 | 5,0 | 4,9 |
| A4 | H4 | 5,4 | 4,9 | 4,7 | 4,5 |
| A5 | H5 | 6,0 | 5,5 | 5,6 | 5,3 |
| A6 | H7 | 5,8 | 4,6 | 4,6 | 3,8 |

## Patentansprüche

1. Benzimidazole der Formel I 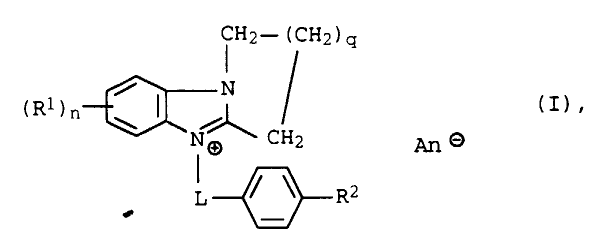 in der
n und q unabhängig voneinander jeweils 1 oder 2,
R¹ Wasserstoff, Chlor oder Methyl,
R² Wasserstoff, gegebenenfalls durch Hydroxy oder C₁-C₄-Alkoxy substituiertes C₁-C₄-Alkyl, Halogen, Nitro oder C₁-C₄-Alkanoyl,
L C₂-C₆-Alkylen und
An^{⊖} das Äquivalent eines Anions bedeuten.

2. Benzimidazole nach Anspruch 1, dadurch gekennzeichnet, daß
q 1,
R¹ Wasserstoff,
R² Wasserstoff oder C₁-C₄-Alkyl und
L C₃-C₆-Alkylen bedeuten.

3. Elektrostatische Toner, enthaltend ein polymeres Bindemittel und als Ladungsstabilisator ein Benzimidazol gemäß Anspruch 1.

4. Verwendung der Benzimidazole gemäß Anspruch 1 als Ladungsstabilisatoren in elektrostatischen Tonern.

## Claims

1. A benzimidazole of the formula I 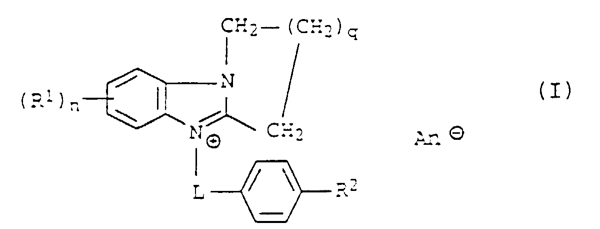 where n and q independently of one another are each 1 or 2, R¹ is hydrogen, chlorine or methyl, R² is hydrogen, unsubstituted or hydroxyl- or C₁-C₄-alkoxy-substituted C₁-C₄-alkyl, halogen, nitro or C₁-C₄-alkanoyl, L is C₂-C₆-alkylene and An^{⊖} is one equivalent of an anion.

2. A benzimidazole as claimed in claim 1, wherein q is 1, R¹ is hydrogen, R² is hydrogen or C₁-C₄-alkyl and L is C₃-C₆-alkylene.

3. An electrostatic toner containing a polymeric binder and, as a charge stabilizer, a benzimidazole as claimed in claim 1.

4. Use of a benzimidazole as claimed in claim 1 as a charge stabilizer in electrostatic toners.

## Revendications

1. Benzimidazoles de formule I 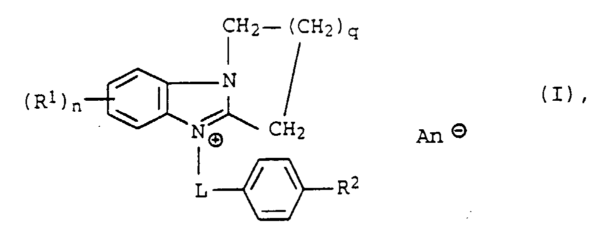 dans laquelle
n et q sont mis chacun, indépendamment l'un de l'autre, pour 1 ou 2,
R¹ représente un atome d'hydrogène, de chlore ou un reste méthyle,
R² représente un atome d'hydrogène, un reste alkyle en C₁-C₄ éventuellement substitué par un groupement hydroxy ou alcoxy en C₁-C₄, un atome d'halogène, un reste nitro ou alcanoyle en C₁-C₄,
L représente un reste alkylène en C₂-C₆ et
An^{⊖} est l'équivalent d'un anion.

2. Benzimidazoles selon la revendication 1, caractérisés en ce que
q est mis pour 1,
R¹ représente un atome d'hydrogène,
R² représente un atome d'hydrogène ou un reste alkyle en C₁-C₄ et
L représente un reste alkylène en C₃-C₆.

3. Toner électrostatique, contenant un liant polymère et, en tant que stabilisateur de charge, un benzimidazole selon la revendication 1.

4. Utilisation des benzimidazoles selon la revendication 1 comme stabilisateurs de charge dans des toners électrostatiques.
